# EUROPÄISCHE PATENTSCHRIFT

(11) **EP 2 405 860 B1**
(45) Veröffentlichungstag und Bekanntmachung des Hinweises auf die Patenterteilung: **25.09.2013**
(21) Anmeldenummer: 10709996.2
(22) Anmeldetag: 11.03.2010
(51) Int. Cl.: A61F 2/14, C08F 220/28, C08F 220/30, G02B 1/04

(54) **OPHTHALMOLOGISCHE ZUSAMMENSETZUNG UND OPHTHALMOLOGISCHE LINSE**
OPHTHALMOLOGICAL COMPOSITION AND OPHTHALMOLOGICAL LENS
COMPOSITION OPHTALMOLOGIQUE ET LENTILLE OPHTALMOLOGIQUE

(30) Priorität: 12.03.2009 DE 102009012959
(43) Veröffentlichungstag der Anmeldung: 18.01.2012
(73) Patentinhaber: Carl Zeiss Meditec AG, 07745 Jena (DE)
(72) Erfinder: RITTER, Helmut, 42111 Wuppertal (DE); SCHMITZ, Daniel, 22529 Hamburg (DE)
(74) Vertreter: Hofstetter, Schurack & Partner
(86) Internationale Anmeldenummer: PCT/EP2010/053139
(87) Internationale Veröffentlichungsnummer: WO 2010/103089

(56) Entgegenhaltungen:
- EP-A2- 0 231 572
- WO-A2-2009/074520
- GB-A- 1 604 519

## Beschreibung

### Technisches Gebiet

Die vorliegende Erfindung betrifft eine ophthalmologische Zusammensetzung und eine ophthalmologische Linse.

### Stand der Technik

Es ist bekannt, ophthalmologische Linsen wie beispielsweise Intraokularlinsen (IOL) einteilig oder mehrteilig auszubilden. Bei einteiligen IOLs bestehen der optische und der nichtoptische Bereich aus einem Werkstoff. Bei mehrteiligen IOLs können der optische und der nichtoptische Bereich aus verschiedenen Werkstoffen bestehen. Der nichtoptische Bereich wird auch als haptischer Teil bezeichnet und dient zur Befestigung der Linse im Auge eines Patienten.

Um eine Verringerung der Länge des zum Einführen der ophthalmologischen Linse ins Auge erforderlichen Schnittes zu erzielen, ist es dabei wünschenswert, Polymere bzw. ophthalmologische Zusammensetzungen zur Verfügung zu stellen, welche z.B. aufgrund ihrer Flexibilität und Entfaltungseigenschaften eine Verkürzung des Einschnittes ermöglichen. Somit sollte ein Polymer, aus dem eine ophthalmologische Linse hergestellt wird, diesbezüglich vorteilhafte Eigenschaften aufweisen.

Die EP 0 231 572 A2 offenbart sauerstoffdurchlässige harte und mittelharte Kontaktlinsen, die aus Copolymeren ethylenisch ungesättigter Siloxanester, ethylenisch ungesättiger Fluorcarbonester und ethylenisch ungesättigter Sulfonmonomere bestehen.

Aus der GB 1 604 519 A sind Kontaktlinsen aus Polysiloxanpolymeren bekannt.

In der WO 2005/047349 A1 wird eine einteilige Intraokularlinse mit einem optischen und einem haptischen Teil vorgeschlagen, bei welcher der optische und der haptische Bereich aus demselben Polymer hergestellt werden. Das Polymer ist dabei als Copolymer ausgebildet und weist neben Alkoxyalkylmethacrylat- und Alkoxyalkylacrylat-Monomeren weitere Acrylat- und Methacrylat-Monomere als Untereinheiten auf, von denen bezogen auf das Gesamtgewicht des Copolymers maximal 55% im Copolymer enthalten sein können.

Als nachteilig an den bekannten Polymeren ist jedoch der Umstand anzusehen, dass diese entweder eine eingeschränkte Faltbarkeit oder einen vergleichsweise geringen Brechungsindex aufweisen, wodurch in beiden Fällen relativ große Einschnitte beim Implantieren von aus diesen Polymeren hergestellten ophthalmologischen Linsen und Implantaten erforderlich sind.

### Darstellung der Erfindung

Die Aufgabe der vorliegenden Erfindung ist es, ein Polymer sowie eine entsprechende ophthalmologische Linse bereitzustellen, das bzw. die verbesserte mechanische und optische Eigenschaften bei gleichzeitig hoher Biokompatibilität aufweist bzw. aufweisen.

Diese Aufgabe wird erfindungsgemäß durch eine ophthalmologische Zusammensetzung gemäß Patentanspruch 1 sowie durch eine ophthalmologische Linse gemäß Patentanspruch 11 gelöst. Vorteilhafte Ausgestaltungen mit zweckmäßigen und nicht-trivialen Weiterbildungen der Erfindung sind in den jeweiligen Unteransprüchen angegeben, wobei vorteilhafte Ausgestaltungen des Polymers als vorteilhafte Ausgestaltungen der ophthalmologische Zusammensetzung sowie der ophthalmologischen Linse und umgekehrt anzusehen sind.

Ein erfindungsgemäßes Polymer für eine ophthalmologische Zusammensetzung, welches verbesserte mechanische und optische Eigenschaften bei gleichzeitig hoher Biokompatibilität besitzt, umfasst wenigstens ein hydrophobes Monomer der allgemeinen Formel I 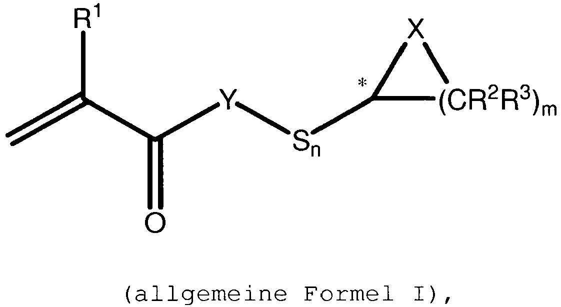 wobei das Polymer eine Wasseraufnahmefähigkeit von höchstens 10% besitzt und R¹, R² und R³ jeweils unabhängig voneinander Wasserstoff oder Alkyl-Reste bedeuten, Y O oder NR⁴ mit R⁴ ausgewählt aus Wasserstoff oder Alkyl-Rest bedeutet, X O, S, SO oder SO₂ bedeutet, S eine Struktureinheit ausgewählt aus CR⁵₂ und/oder (CR⁵₂CR⁵₂O)ₒCR⁵₂ bedeutet, wobei alle R⁵ jeweils unabhängig voneinander Wasserstoff und/oder Alkyl-Reste bedeuten, n und o unabhängig voneinander 1, 2, 3, 4, 5, 6, 7, 8, 9 und/oder 10 und m 2, 3, 4, 5 oder 6 bedeuten. Dabei sind grundsätzlich alle Enantiomere, Diastereomere und racemischen Gemische der allgemeinen Formel I als mitoffenbart anzusehen. Grundsätzlich kann vorgesehen sein, dass das wenigstens eine hydrophobe Monomer der allgemeinen Formel I in enantiomerenreiner Form oder als racemisches Gemisch vorliegt. Überraschenderweise zeigt das Polymer gemäß der vorliegenden Erfindung ein verbessertes mechanisches und optisches Eigenschaftsprofil verglichen mit den Copolymeren des Standes der Technik.

Das erfindungsgemäße Polymer ermöglicht dabei aufgrund seiner Hydrophobizität, seiner geringen Wasseraufnahmefähigkeit und seines hohen Brechungsindex eine Herstellung von ophthalmologischen Linsen, Implantaten und dergleichen, welche im Vergleich zum Stand der Technik einen höheren Brechungsindex aufweisen, da sich dieser aus der nach Massenanteil gewichteten Summe der Brechungsindices des wasserfreien Polymers und des von diesem aufgenommenen Wassers ergibt, wobei Wasser lediglich einen Brechungsindex von 1,3 aufweist. Da das Polymer einerseits eine hohe Brechkraft und andererseits eine geringe Wasseraufnahmefähigkeit besitzt, können aus dem erfindungsgemäßen Polymer Linsen, Implantate und dergleichen hergestellt werden, die im Vergleich zu Linsen aus bekannten Materialien bei gleicher Dioptrie eine geringere Wandstärke besitzen.

Da auch die Roll- und Faltbarkeit von ophthalmologischen Linsen und Implantaten wesentlich von der Wandstärke abhängt, können Implantate, die zumindest teilweise aus dem erfindungsgemäßen Polymer bestehen, entsprechend enger aufgerollt oder gefaltet werden. Der Einschnitt bei der Implantation kann somit deutlich verkürzt werden. Darüber hinaus ist die biologische Verträglichkeit des erfindungsgemäßen Polymers im Auge aufgrund seiner geringen Wasseraufnahmefähigkeit und der damit verringerten Wechselwirkung mit Körpergewebe sowie aufgrund der vorteilhaften biologischen und chemischen Eigenschaften des hydrophoben Monomers vorteilhaft verbessert. Das Polymer weist darüber hinaus eine optimale Verarbeitbarkeit für die Herstellung einer ophthalmischen Linse, eines Implantats und dergleichen auf, da sich die Glasübergangstemperatur des Polymers durch Variierung des wenigstens einen hydrophoben Monomers in einem breiten Bereich gezielt einstellen lässt. Dabei ist zu betonen, dass das Polymer in einfachster Ausgestaltung als Homopolymer ausgebildet sein kann und damit lediglich einen Monomertyp der allgemeinen Formel I umfasst.

In einer vorteilhaften Ausgestaltung der Erfindung ist vorgesehen, dass das Polymer eine Wasseraufnahmefähigkeit von höchstens 5%, insbesondere von höchstens 2% und vorzugsweise von höchstens 1% besitzt. Hierdurch kann einerseits der Brechungsindex des Polymers zusätzlich vergrößert werden, wodurch sich andererseits auch dessen biologische Verträglichkeit aufgrund der zusätzlich verringerten Wechselwirkung mit biologischem Gewebe weiter verbessert. Weiterhin können aus dem erfindungsgemäßen Polymer besonders gut falt- und rollbare Linsen, Implantate etc. hergestellt werden, die dementsprechend kleine Einschnitte bei der Implantation erfordern.

Weitere Vorteile ergeben sich, wenn ein Gewichtsanteil des wenigstens einen hydrophoben Monomers der allgemeinen Formel I bezogen auf das Gesamtgewicht des Polymers zwischen 2 und 100 Gewichtsprozent beträgt. Die im Kontext der Offenbarung angegebenen Monomer-Anteile beziehen sich grundsätzlich auf das Gesamtgewicht des Polymers und sind unter Berücksichtigung eines etwaigen Wasseranteils so zu wählen, dass in der Summe 100 Gewichtsprozent erhalten werden. Für den Fall, dass weitere Inhaltsstoffe wie Radikalstarter oder dergleichen in dem Polymer enthalten sind, sind die Gewichtsanteile so zu wählen, dass sich ein Gesamtgewicht des Polymers inklusive der weiteren Inhaltsstoffe entsprechend zu 100 Gewichtsprozent ergibt.

In einer weiteren vorteilhaften Ausgestaltung der Erfindung ist vorgesehen, dass R¹ und/oder R² und/oder R³ und/oder R⁴ und/oder R⁵ Wasserstoff und/oder ein unverzweigter und/oder verzweigter Alkyl-Rest mit vorzugsweise 1, 2, 3, 4, 5, 6, 7, 8, 9 und/oder 10 Kohlenstoffatomen ist. Hierdurch können die mechanischen und optischen Eigenschaften des Polymers besonders einfach, variabel und präzise an das jeweilige Anforderungsprofil angepasst werden. Als Alkyl-Rest können dabei bevorzugt Methyl-, Ethyl-, n-Propyl-, iso-Propyl-, n-Butyl-, iso-Butylund/oder tert-Butyl-Reste vorgesehen sein. Alternativ oder zusätzlich können jedoch auch Pentyl-, Hexyl-, Heptyl-, Octyl-, Nonyl- oder Decyl-Reste bzw. deren Stellungsisomere mit einer entsprechenden Gesamtkohlenstoffanzahl vorgesehen sein, um die mechanischen und optischen Eigenschaften des Polymers gezielt einzustellen.

Weitere Vorteile ergeben sich, indem R¹ Wasserstoff oder CH₃- ist, wenn Y O ist, oder dass R¹ Wasserstoff ist, wenn Y NH ist. Hierdurch besitzt das Polymer eine besonders hohe Faltbarkeit und entsprechend vorteilhafte Eignung für Kleinschnittanwendungen.

In einer weiteren vorteilhaften Ausgestaltung der Erfindung ist vorgesehen, dass das Monomer der allgemeinen Formel I Tetrahydrofufurylmethacrylat ((Tetrahydrofuran-2-yl)methylmethacrylat, THFFMA) und/oder Tetrahydrofufurylacrylat ((Tetrahydrofuran-2-yl)methylacrylate, THFFA) ist. Hierdurch besitzt das Polymer besonders vorteilhafte Eigenschaften, indem neben einer besonders hohen Faltbarkeit auch eine hohe chemische Beständigkeit gegenüber Umwelteinflüssen sowie eine besonders gute Biokompatibilität gegeben sind. Dabei kann vorgesehen sein, dass das Polymer - gegebenenfalls neben Radikalstartern, Vernetzern oder dergleichen - ausschließlich THFFMA und/oder THFFA als Monomere umfasst.

In weiterer vorteilhafter Ausgestaltung ist vorgesehen, dass das Polymer zumindest ein weiteres hydrophobes Monomer umfasst, welches somit nicht der allgemeinen Formel I entspricht. Hierdurch ist insbesondere eine besonders variable und präzise Einstellbarkeit der Glasübergangstemperatur des Polymers ermöglicht. Grundsätzlich kann auch in diesem Fall vorgesehen sein, dass das wenigstens eine weitere hydrophobe Monomer in enantiomerenreiner Form oder als racemisches Gemisch vorliegt. Das weitere hydrophobe Monomer kann beispielsweise aus der Gruppe der unverzweigten Alkyl(meth)acrylate gewählt sein. Beispielhafte Vertreter sind hierbei Methyl(meth)acrylat, Ethyl(meth)acrylat, Propyl(meth)acrylat, Butyl(meth)-acrylat, Hexyl(meth)acrylat und Lauryl(meth)acrylat. Alternativ oder zusätzlich können auch verzweigte und/oder cyclische Monomere als weiteres hydrophobes Monomer vorgesehen sein. Eine weitere Gruppe geeigneter hydrophober Monomere stellen die Alkoxyalkoxyalkyl(meth)acrylate dar, von denen sich beispielsweise Ethoxyethoxyethyl(meth)acrylat und/oder Cyclohexyl(meth)acrylat als geeignet gezeigt haben. Es ist jedoch zu betonen, dass die genannten Gruppen und Verbindungen als lediglich beispielhafte und somit nicht-abschließende Aufzählung zu verstehen sind.

In einer weiteren vorteilhaften Ausgestaltung der Erfindung ist vorgesehen, dass das zumindest eine weitere hydrophobe Monomer die allgemeinen Formel II 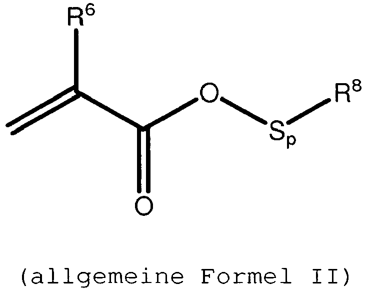 besitzt, wobei S ein Strukturelement ausgewählt aus CR⁷₂ und/oder (CR⁸₂CR⁸₂O)ₒCR⁸₂ bedeutet. Alle R⁷ bedeuten dabei unabhängig voneinander Wasserstoff, Alkyl-Reste und/oder Cycloalkyl-Reste. Weiterhin bedeuten alle R⁸ unabhängig voneinander Wasserstoff und/oder Alkyl-Reste und p und o unabhängig voneinander eine ganze Zahl zwischen 1 und 10. Dabei sind auch beim weiteren hydrophoben Monomer der allgemeinen Formel II grundsätzlich alle Diastereomere, Enantiomere oder racemischen Gemische als mitoffenbart anzusehen. Mit Hilfe eines Monomers der allgemeinen Formel II ist eine zusätzliche Möglichkeit gegeben, die chemischen, mechanischen und optischen Eigenschaften des Polymers optimal an den jeweiligen Einsatzzweck anzupassen. Dabei hat es sich als vorteilhaft gezeigt, wenn die Reste R⁶, R⁷ und R⁸ jeweils unabhängig voneinander ausgewählt aus unverzweigten und/oder verzweigten und/oder cyclischen Alkyl-Gruppen mit vorzugsweise 1, 2, 3, 4, 5, 6, 7, 8, 9 und/oder 10 Kohlenstoffatomen gewählt sind. Die Reste R⁶, R⁷ und R⁸ können daher beispielsweise eine Methyl-, Ethyl-, n-Propyl-, iso-Propyl-, n-Butyl-, iso-Butyl-, tert-Butyl- und/oder Cyclohexyl-Gruppe sein. Alternativ oder zusätzlich können jedoch auch Pentyl-, Hexyl-, Heptyl-, Octyl-, Nonyl- oder Decyl-Reste bzw. deren Stellungsisomere mit einer entsprechenden Gesamtkohlenstoffanzahl vorgesehen sein, um die mechanischen und optischen Eigenschaften des Polymers gezielt einzustellen. Vorzugsweise ist R⁶ Wasserstoff oder CH₃-, um ein Monomer auf Acrylat- bzw. Methacrylat-Basis zu erhalten.

Weitere Vorteile ergeben sich, indem ein Gewichtsanteil des zumindest einen weiteren hydrophoben Monomers bezogen auf das Gesamtgewicht des Polymers zwischen 1 und 55 Gewichtsprozent beträgt. Hierdurch kann der Gewichtsanteil des wenigstens einen weiteren hydrophoben Monomers optimal in Abhängigkeit der Art und Menge des wenigstens einen hydrophoben Monomers der allgemeinen Formel I gewählt werden, um die gewünschten Eigenschaften des Polymers zu erzielen.

Indem das Polymer einen UV-Absorber umfasst, kann ein optimaler Schutz des Polymers und - bei Verwendung des Polymers für ophthalmologische Anwendungen - des Auges eines Patienten vor kurz- und langwelligem UV-Licht sichergestellt werden. Als UV-Absorber eignen sich insbesondere Verbindungen, die Strahlung im Wellenlängenbereich zwischen etwa 200 nm und etwa 420 nm vorzugsweise überwiegend quantitativ und besonders bevorzugt quantitativ absorbieren. Aufgrund der Eigenschaften des wenigstens einen hydrophoben Monomers der allgemeinen Formel I weist der UV-Absorber darüber hinaus im Unterschied zum Stand der Technik eine deutlich erhöhte Lichtbeständigkeit und Haltbarkeit auf.

Dabei hat es sich in weiterer Ausgestaltung als vorteilhaft gezeigt, wenn der UV-Absorber die allgemeine Formel III 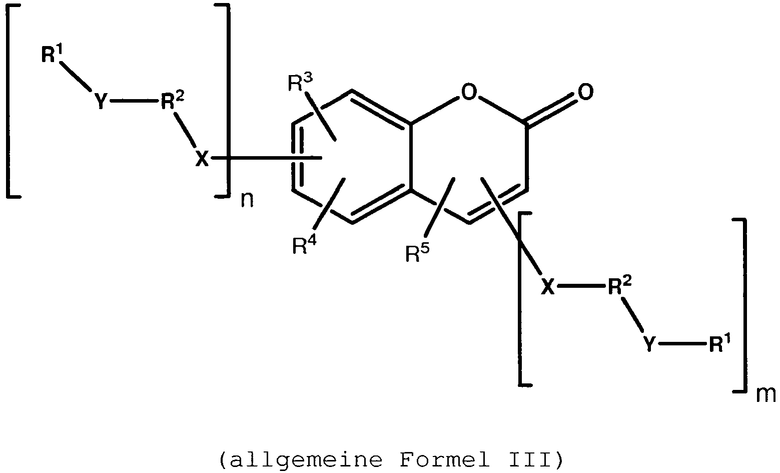 besitzt, wobei R¹ unabhängig voneinander Acryl- und/oder Methacryl-Reste bedeuten, R² unabhängig voneinander verzweigte und/oder unverzweigte Alkyl- und/oder Aryl-Reste mit bis zu 30 Atomen ausgewählt aus C, H, Si, O, N, P, S, F, Cl, Br bedeuten, R³, R⁴ und R⁵ unabhängig voneinander Wasserstoff und/oder verzweigte und/oder unverzweigte Alkyl- und/oder Aryl-Reste mit bis zu 30 Atomen ausgewählt aus C, H, Si, O, N, P, S, F, Cl, Br bedeuten, X und Y unabhängig voneinander O, S, NH oder NR⁶ bedeuten, wobei R⁶ ein verzweigter und/oder unverzweigter Alkyl- und/oder Aryl-Rest mit bis zu 30 Atomen ausgewählt aus C, H, Si, O, N, P, S, F, Cl, Br ist, und n=0 bis 2 sowie m=0 oder 1 bedeuten, wobei n+m immer größer gleich 1 ist. Nicht limitierende Beispiele vorteilhafter Strukturen (mit R = Wasserstoff und/oder CH₃; alle Stereoisomere oder racemischen Gemische sind beinhaltet) sind: 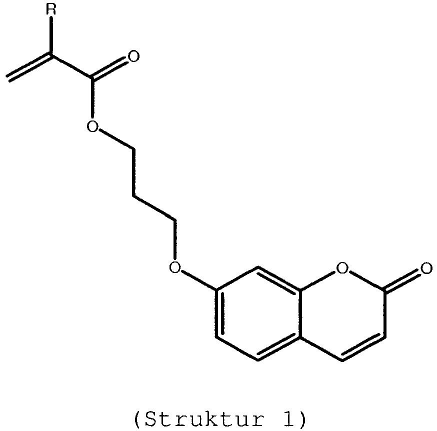 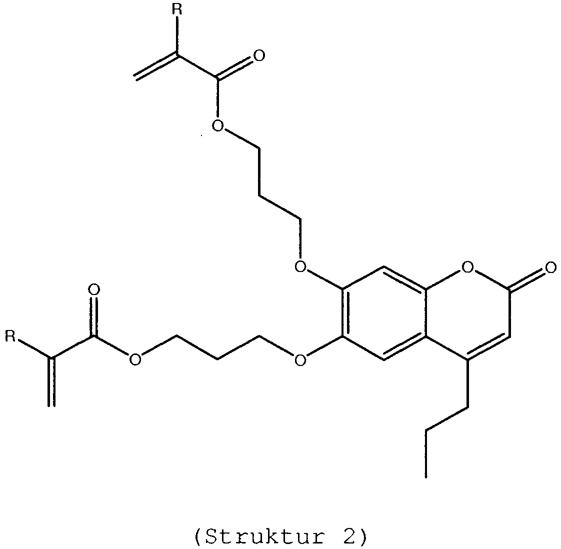 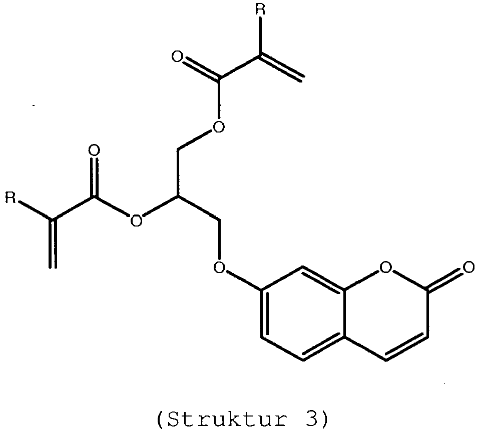 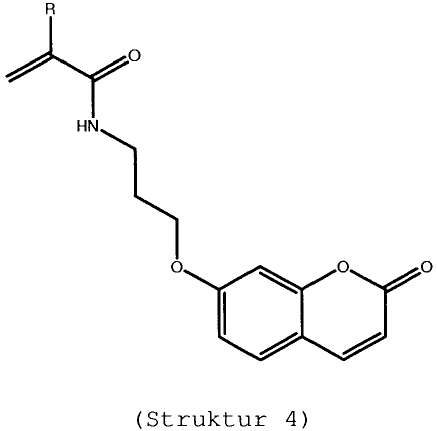 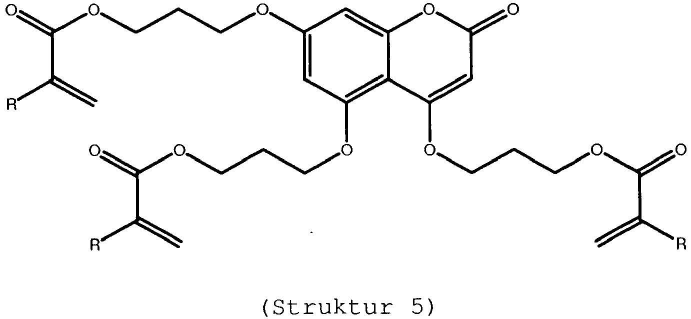 UV-Absorber, deren Grundstruktur auf den Strukturen 2, 3 und 5 basieren, haben den zusätzlichen Vorteil, dass diese bedingt durch das Vorhandensein mehrerer polymerisierbarer Endgruppen nicht nur einen quantitativen Einbau in das Polymer ermöglichen, sondern darüber hinaus auch vernetzende Eigenschaften besitzen. Hierdurch kann auf die Zugabe eines zusätzlichen Vernetzers verzichtet werden.

Weitere Vorteile ergeben sich, wenn das Polymer wenigstens einen Violett-Absorber umfasst. Vorzugsweise absorbiert der Violett-Absorber (sog. Gelbfarbstoff) vorzugsweise im Wesentlichen quantitativ oder besonders bevorzugt quantitativ violettes Licht im Wellenlängenbereich zwischen etwa 400 nm und etwa 430 nm. Es können grundsätzlich jedoch auch zwei oder mehr unterschiedliche Violett-Absorber mit unterschiedlichen Absorptionscharakteristika vorgesehen sein. Besonders bevorzugt handelt es sich bei dem wenigstens einen Violett-Absorber um einen Kantenfilter, d.h. dass dieser Licht der Wellenlängen von etwa 400 nm bis 430 nm quantitativ absorbiert und nachfolgend einen starken Anstieg der Transmission hin zu größeren Wellenlängen aufweist. Beispielsweise kann der Violett-Absorber derart ausgebildet sein, dass er im Wellenlängenbereich zwischen etwa 400 nm und 430 nm einen Transmissionsgrad von höchstens 0,05 und im Wellenlängenbereich zwischen etwa 490 nm und etwa 550 nm einen Transmissionsgrad von mindestens 0,90 besitzt.

Ein derartiges Absorptionsprofil ist in weiterer vorteilhafter Ausgestaltung der Erfindung durch einen Violett-Absorber mit der allgemeinen Formel IV 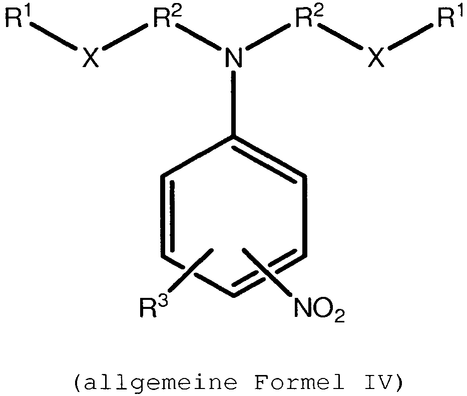 bereitgestellt, wobei R¹ unabhängig voneinander Acrylund/oder Methacryl-Reste bedeuten, R² unabhängig voneinander verzweigte und/oder unverzweigte Alkyl- und/oder Aryl-Reste mit bis zu 30 Atomen ausgewählt aus C, H, Si, O, N, P, S, F, Cl, Br bedeuten, R³ Wasserstoff, elektronenziehender Substituent oder verzweigter und/oder unverzweigter Alkyl- und/oder Aryl-Rest mit bis zu 30 Atomen ausgewählt aus C, H, Si, O, N, P, S, F, Cl, Br bedeutet, und X O, S, NH oder NR⁴ bedeutet, wobei R⁴ ein verzweigter und/oder unverzweigter Alkyl- und/oder Aryl-Rest mit bis zu 30 Atomen ausgewählt aus C, H, Si, O, N, P, S, F, Cl, Br ist) ist. Unter einem elektronenziehenden Substituenten ist dabei eine funktionelle Gruppe zu verstehen, die einen -I-Effekt, d.h. einen negativen induktiven Effekt über eine σ-Bindung ausüben kann und/oder einen -M-Effekt, d.h. einen negativen mesomeren Effekt über eine n-Bindung besitzt. Nicht-limitierende Beispiele geeigneter elektronenziehender Substituenten sind Halogene, Nitro- oder Carbonsäure-Gruppen.

Alternativ oder zusätzlich kann die gewünschte Violett-Absorption dadurch gewährleistet werden, dass der Violett-Absorber die allgemeine Formel V 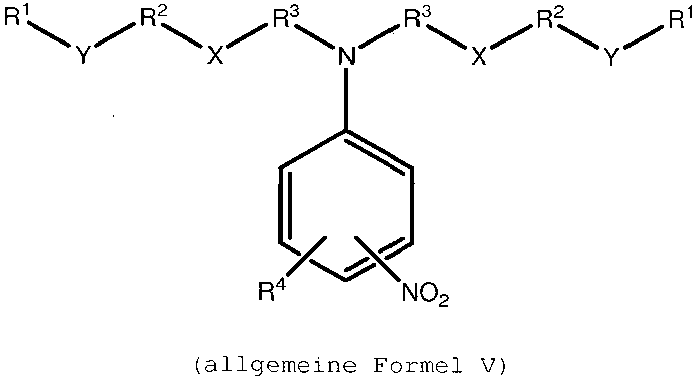 besitzt, wobei R¹ unabhängig voneinander Acryl- und/oder Methacryl-Reste bedeuten, R² und R³ unabhängig voneinander verzweigte und/oder unverzweigte Alkyl- und/oder Aryl-Reste mit bis zu 30 Atomen ausgewählt aus C, H, Si, O, N, P, S, F, Cl, Br bedeuten, R⁴ Wasserstoff, elektronenziehender Substituent oder verzweigter und/oder unverzweigter Alkyl- und/oder Aryl-Rest mit bis zu 30 Atomen ausgewählt aus C, H, Si, O, N, P, S, F, Cl, Br bedeutet, und X O, S, NH oder NR⁵ bedeutet, wobei R⁵ ein verzweigter und/oder unverzweigter Alkyl- und/oder Aryl-Rest mit bis zu 30 Atomen ausgewählt aus C, H, Si, O, N, P, S, F, Cl, Br ist.

Weitere Vorteile ergeben sich, wenn das Polymer zumindest einen Vernetzer umfasst. Mit Hilfe des Vernetzers können Polymerstränge des Polymers zu einem dreidimensionalen Netzwerk verknüpft werden, wodurch das Eigenschaftsprofil des Polymers gezielt verändert und in Abhängigkeit des gewünschten Einsatzzwecks eingestellt werden kann.

Indem zumindest ein Vernetzer ein UV-Absorber und/oder ein Violett-Absorber ist, kann die Anzahl und Menge der zur Herstellung des Polymers eingesetzten Chemikalien vorteilhaft verringert werden, wodurch entsprechende Kostensenkungen erzielbar sind. Darüber hinaus ist der UV-und/oder Violett Absorber auf diese Weise durch kovalente Bindung Medialbestandteil des Polymers, so dass eine Freisetzung des entsprechenden Absorbers in vielen Anwendungen minimiert oder verunmöglicht wird.

In weiterer Ausgestaltung hat es sich als vorteilhaft gezeigt, wenn das Polymer einen Brechungsindex von wenigstens 1,3 und vorzugsweise von wenigstens 1,5 aufweist. Insbesondere bei Verwendung des Polymers für ophthalmologische Implantate sind hierdurch vorteilhafte optische Abbildungseigenschaften sichergestellt.

Indem das Polymer eine Glasübergangstemperatur im Bereich zwischen -10°C und 30°C, insbesondere zwischen 0°C und 30°C und/oder zwischen 5°C und 20°C, besitzt, kann die Verformungsfähigkeit des Polymers insbesondere im Hinblick auf die menschliche oder tierische Körpertemperatur optimal angepasst werden.

Ein weiterer Aspekt der Erfindung betrifft eine ophthalmologische Zusammensetzung, welche erfindungsgemäß verbesserte mechanische und optische Eigenschaften bei gleichzeitig hoher Biokompatibilität aufweist, indem sie ein erfindungsgemäßes Polymer oder eine vorteilhafte Ausführung umfasst. Die sich hieraus ergebenden Merkmale und deren Vorteile sind den vorhergehenden Beschreibungen zu entnehmen und gelten entsprechend für die ophthalmologische Zusammensetzung. Insbesondere ist als eine ophthalmologische Zusammensetzung eine chemische Zusammensetzung oder Zubereitung für die Ophthalmologie zu verstehen.

In einer vorteilhaften Ausgestaltung der Erfindung ist vorgesehen, dass die ophthalmologische Zusammensetzung ausschließlich aus dem Polymer nach einem der vorhergehenden Ausführungsbeispiele besteht. Durch den Verzicht auf zusätzliche Inhaltsstoffe kann die ophthalmologische Zusammensetzung besonders einfach und kostengünstig hergestellt werden, wobei die vorteilhaften Eigenschaften des Polymers vollständig erhalten bleiben.

Ein weiterer Aspekt der Erfindung betrifft eine ophthalmologische Linse, welche erfindungsgemäß verbesserte mechanische und optische Eigenschaften bei gleichzeitig hoher Biokompatibilität aufweist, indem sie ein Polymer nach einem der vorhergehenden Ausführungsbeispiele und/oder eine ophthalmologische Zusammensetzung nach einem der vorhergehenden Ausführungsbeispiele umfasst. Die erfindungsgemäße Linse verfügt hierdurch über einen hohen Brechungsindex sowie über eine besonders gute Faltbarkeit bei hoher Biokompatibilität und kann grundsätzlich einteilig oder mehrteilig ausgebildet sein. Dabei kann grundsätzlich vorgesehen sein, dass die Linse ausschließlich aus dem Polymer bzw. der ophthalmologischen Zusammensetzung besteht. Weitere Merkmale und deren Vorteile sind den vorhergehenden Beschreibungen zu entnehmen und gelten entsprechend für die ophthalmologische Linse.

Dabei hat es sich in einer vorteilhaften Ausgestaltung der Erfindung als vorteilhaft gezeigt, wenn die ophthalmologische Linse als Intraokularlinse oder als Kontaktlinse ausgebildet ist.

In einer weiteren vorteilhaften Ausgestaltung der Erfindung ist vorgesehen, dass die ophthalmologische Linse einen Wassergehalt von höchstens 10%, vorzugsweise von höchstens 5%, bevorzugt von höchstens 2% und besonders bevorzugt von höchstens 1% aufweist.

Ein weiterer Aspekt der Erfindung betrifft ein medizinisches Implantat, insbesondere ein Augenimplantat, welches erfindungsgemäß verbesserte mechanische und optische Eigenschaften bei gleichzeitig hoher Biokompatibilität besitzt, indem es ein Polymer nach einem der vorhergehenden Ausführungsbeispiele und/oder eine ophthalmologische Zusammensetzung nach einem der vorhergehenden Ausführungsbeispiele umfasst. Das erfindungsgemäße Implantat, welches beispielsweise als Linse und/oder als Ring und/oder als Stützelement und/oder als Halteelement und/oder als Prothese ausgebildet sein kann, verfügt hierdurch über eine besonders gute Faltbarkeit bei hoher Biokompatibilität und kann grundsätzlich einteilig oder mehrteilig ausgebildet sein. Dabei kann grundsätzlich vorgesehen sein, dass das Implantat ausschließlich aus dem Polymer bzw. der ophthalmologischen Zusammensetzung besteht. Weitere Merkmale und deren Vorteile sind den vorhergehenden Beschreibungen zu entnehmen und gelten entsprechend für das erfindungsgemäße Implantat.

Ein weiterer Aspekt der Erfindung betrifft eine Verwendung eines Polymers nach einem der vorhergehenden Ausführungsbeispiele und/oder einer ophthalmologischen Zusammensetzung nach einem der vorhergehenden Ausführungsbeispiele zur Herstellung einer ophthalmologischen Linse, insbesondere einer Kontakt- oder Intraokularlinse, und/oder eines medizinischen Implantats. Die sich hieraus ergebenden Merkmale und deren Vorteile sind den vorhergehenden Beschreibungen zu entnehmen und gelten entsprechend für die erfindungsgemäße Verwendung.

### Ausführungsbeispiele der Erfindung

### Ausführungsbeispiel 1

5,4 g Tetrahydrofufurylacrylat (THFFA) werden mit 1,6 g Tetrahydrofufurylmethacrylat (THFFMA), 70 mg Ethylenglykoldimethacrylat (EGDMA) als Vernetzer, 105 mg Cumarin-5,7-di(propoxymethacrylat) als UV-Absorber und 11,2 mg 4-Nitrophenylimino-di(2'-ethylmethacrylat) als Violett-Absorber unter starkem Rühren bei 35°C gemischt. Die Reaktionsmischung wird mit Stickstoff entgast und für etwa 10 Minuten gerührt. Die Polymerisation wird durch Zugabe von 4,9 mg 2,2'-Azobis(2,4-dimethylvaleronitril) eingeleitet und die Mischung mit einer Spritze in eine geeignete Polymerisationsform überführt. Die Polymerisation der Mischung erfolgt über einen Zeitraum von 24 Stunden bei 60°C. Nach Ablauf der Polymerisation wird das Polymer (sogenannter Copolymer-Blank) aus den Formen entnommen und für 48 Stunden bei 125°C und 1,1 mbar im Vakuumtrockenschrank thermisch nachbehandelt. Das erhaltene Polymer, welches abgesehen vom Vernetzer, den UV- und Violett-Absorbern und dem Radikalstarter ausschließlich hydrophobe Monomere der allgemeinen Formel I umfasst, weist eine Glasübergangstemperatur Tg von etwa 5°C sowie einen Brechungsindex von etwa 1,5 auf und kann in an sich bekannter Weise zur Herstellung von medizinischen Implantaten, insbesondere zur Herstellung ophthalmologischer Linsen verwendet werden.

### Ausführungsbeispiel 2

Das Polymer gemäß dem zweiten Ausführungsbeispiel wurde ebenfalls mit Hilfe des im ersten Ausführungsbeispiel beschriebenen Verfahrens hergestellt. Die vorliegend verwendeten Edukte sowie die Verfahrensparameter sind in Tabelle 1 angegeben. Im Unterschied zum ersten Ausführungsbeispiel wurde kein zusätzlicher Vernetzer verwendet, da sowohl der UV-Absorber (Cumarin-5,7-di(propoxymethacrylat)), als auch der Violett-Absorber (4-Nitrophenylimino-bis-(2'-ethylmethacrylat)) zwei polymerisierbare Endgruppen aufweisen und somit vernetzende Eigenschaften besitzen. Auch das Polymer gemäß dem zweiten Ausführungsbeispiel umfasst somit abgesehen vom Radikalstarter (V65) und den UV- und Violett-Absorbern ausschließlich hydrophobe Monomere der allgemeinen Formel I.

**Tabelle 1**

| | |
|---|---|
| Gesamteinwaage [g] | 7, 0 |
| Tetrahydrofufurylmethacrylat | |
| Einwaage [g] | 1,376 |
| Gew.% (soll) | 20,00 |
| Gew.% (ist) | 19, 65 |
| Tetrahydrofufurylacrylat | |
| Einwaage [g] | 5,503 |
| Gew.% (soll) | 80, 00 |
| Gew.% (ist) | 78, 62 |
| Radikalstarter V65 | |
| Einwaage [g] | 0, 0049 |
| Gew.% | 0,07 |
| Cumarin-5,7-di(propoxymethacrylat) | |
| Einwaage [g] | 0,105 |
| Gew.% | 1,5 |
| 4-Nitrophenylimino-bis-(2'-ethylmethacrylat) | |
| Einwaage [g] | 0, 0112 |
| Gew.% | 0,16 |
| Trockenschrank 70°C [h] | 24 |
| Vakuumtrockenschrank 125°C; 5,5*10⁻² mbar [h] | 48 |
| Glasübergangstemperatur Tg [°C] | 5,1 |
| Brechungsindex | 1,5 |

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, Einwaagefehlern, DIN-Toleranzen und dergleichen - als vom Rahmen der Erfindung mitumfasst anzusehen.

## Patentansprüche

1. Ophthalmologische Zusammensetzung mit einem Polymer, welches wenigstens ein hydrophobes Monomer der allgemeinen Formel I 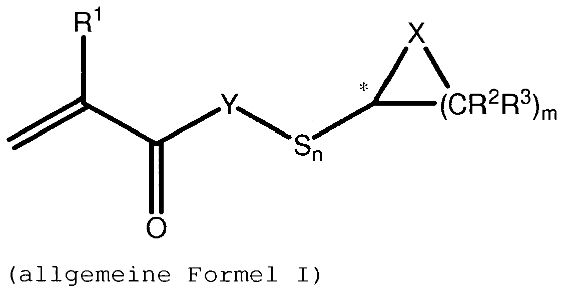 umfasst,
wobei das Polymer eine Wasseraufnahmefähigkeit von höchstens 10% besitzt und:
R¹ R² und R³ jeweils unabhängig voneinander Wasserstoff oder Alkyl-Reste bedeuten;
Y O oder NR⁴ mit R⁴ ausgewählt aus Wasserstoff oder Alkyl-Rest bedeutet;
X O, S, SO oder SO₂ bedeutet;
S eine Struktureinheit ausgewählt aus CR⁵₂ und/oder (CR⁵₂CR⁵₂O)ₒCR⁵₂ bedeutet, wobei alle R⁵ jeweils unabhängig voneinander Wasserstoff und/oder Alkyl-Reste bedeuten;
n und o unabhängig voneinander eine ganze Zahl zwischen 1 und 10; und
m eine ganze Zahl zwischen 2 und 6 bedeuten.

2. Ophthalmologische Zusammensetzung nach Anspruch 1, **dadurch gekennzeichnet, dass** das Polymer eine Wasseraufnahmefähigkeit von höchstens 5%, insbesondere von höchstens 2% und vorzugsweise von höchstens 1% besitzt.

3. Ophthalmologische Zusammensetzung nach Anspruch 1 oder 2, **dadurch gekennzeichnet, dass** ein Gewichtsanteil des wenigstens einen hydrophoben Monomers der allgemeinen Formel I bezogen auf das Gesamtgewicht des Polymers zwischen 2 und 100 Gewichtsprozent beträgt.

4. Ophthalmologische Zusammensetzung nach einem der Ansprüche 1 bis 3, **dadurch gekennzeichnet, dass** R¹ Wasserstoff oder CH₃- ist, wenn Y O ist, oder dass R¹ Wasserstoff ist, wenn Y NH ist.

5. Ophthalmologische Zusammensetzung nach einem der Ansprüche 1 bis 4, **dadurch gekennzeichnet, dass** das Monomer der allgemeinen Formel I Tetrahydrofufurylmethacrylat (THFFMA) und/oder Tetrahydrofufurylacrylat (THFFA) ist.

6. Ophthalmologische Zusammensetzung nach einem der Ansprüche 1 bis 5, **dadurch gekennzeichnet, dass** das Polymer zumindest ein weiteres hydrophobes Monomer umfasst.

7. Ophthalmologische Zusammensetzung nach einem der Ansprüche 1 bis 6, **dadurch gekennzeichnet, dass** das Polymer einen UV-Absorber und/oder wenigstens einen Violett-Absorber umfasst.

8. Ophthalmologische Zusammensetzung nach einem der Ansprüche 1 bis 7, **dadurch gekennzeichnet, dass** das Polymer zumindest einen Vernetzer umfasst, wobei der Vernetzer insbesondere ein UV-Absorber und/oder ein Violett-Absorber ist.

9. Ophthalmologische Zusammensetzung nach einem der Ansprüche 1 bis 8, **dadurch gekennzeichnet, dass** das Polymer einen Brechungsindex von wenigstens 1,3 und vorzugsweise von wenigstens 1,5 aufweist.

10. Ophthalmologische Zusammensetzung nach einem der Ansprüche 1 bis 9, **dadurch gekennzeichnet, dass** das Polymer eine Glasübergangstemperatur im Bereich zwischen -10°C und 30°C, insbesondere zwischen 0°C und 30°C und/oder zwischen 5°C und 20°C, besitzt.

11. Ophthalmologische Linse, umfassend eine ophthalmologische Zusammensetzung nach einem der Ansprüche 1 bis 10.

12. Ophthalmologische Linse nach Anspruch 11, **dadurch gekennzeichnet, dass** diese als Intraokularlinse oder als Kontaktlinse ausgebildet ist.

13. Ophthalmologische Linse nach Anspruch 11 oder 12, **dadurch gekennzeichnet, dass** diese einen Wassergehalt von höchstens 10%, vorzugsweise von höchstens 5%, bevorzugt von höchstens 2% und besonders bevorzugt von höchstens 1% aufweist.

14. Verwendung einer ophthalmologischen Zusammensetzung nach einem der Ansprüche 1 bis 10 zur Herstellung einer ophthalmologischen Linse, insbesondere einer Kontakt- oder Intraokularlinse, und/oder eines medizinischen Implantats.

## Claims

1. Ophthalmologic composition including a polymer comprising at least one hydrophobic monomer of the general formula I 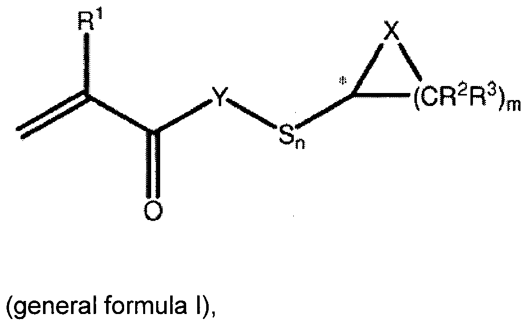 wherein the polymer has a water absorption capacity of at most 10%, and:
R¹, R² and R³ each independently of each other denote hydrogen or alkyl radicals;
Y denotes O or NR⁴ with R⁴ selected from hydrogen or alkyl radical;
X denotes O, S, SO or SO₂;
S denotes a structural unit selected from CR⁵₂ and/or (CR⁵₂CR⁵₂O)ₒCR⁵₂, wherein all R⁵ each independently of each other denote hydrogen and/or alkyl radicals;
n and o independently of each other denote an integer between 1 and 10; and
m denotes an integer between 2 and 6.

2. Ophthalmologic composition according to claim 1, **characterized in that** the polymer has a water absorption capacity of at most 5%, in particular of at most 2% and preferably of at most 1%.

3. Ophthalmologic composition according to claim 1 or 2, **characterized in that** a weight portion of the at least one hydrophobic monomer of the general formula I related to the overall weight of the polymer is between 2 and 100 percent by weight.

4. Ophthalmologic composition according to any one of claims 1 to 3, **characterized in that** R¹ is hydrogen or CH₃- if Y is O, or that R¹ is hydrogen if Y is NH.

5. Ophthalmologic composition according to any one of claims 1 to 4, **characterized in that** the monomer of the general formula I is tetrahydrofufuryl methacrylate (THFFMA) and/or tetrahydrofufuryl acrylate (THFFA).

6. Ophthalmologic composition according to any one of claims 1 to 5, **characterized in that** the polymer includes at least one further hydrophobic monomer.

7. Ophthalmologic composition according to any one of claims 1 to 6, **characterized in that** the polymer includes an UV absorber and/or at least a violet absorber.

8. Ophthalmologic composition according to any one of claims 1 to 7, **characterized in that** the polymer includes at least a cross-linker, wherein the cross-linker is in particular an UV absorber and/or a violet absorber.

9. Ophthalmologic composition according to any one of claims 1 to 8, **characterized in that** the polymer has a refractive index of at least 1.3 and preferably of at least 1.5.

10. Ophthalmologic composition according to any one of claims 1 to 9, **characterized in that** the polymer has a glass transition temperature in the range between -10°C and 30 °C, in particular between 0 °C and 30 °C and/or between 5°C and 20°C.

11. Ophthalmologic lens including an ophthalmologic composition according to any one of claims 1 to 10.

12. Ophthalmologic lens according to claim 11, **characterized in that** it is formed as an intraocular lens or as a contact lens.

13. Ophthalmologic lens according to claim 11 or 12, **characterized in that** it has a water content of at most 10%, preferably of at most 5 %, preferred of at most 2% and particularly preferred of at most 1%.

14. Use of an ophthalmologic composition according to any one of claims 1 to 10 for manufacturing an ophthalmologic lens, in particular a contact or intraocular lens, and/or a medical implant.

## Revendications

1. Composition ophtalmologique avec un polymère, lequel comprend au moins un monomère hydrophobe de la formule générale I 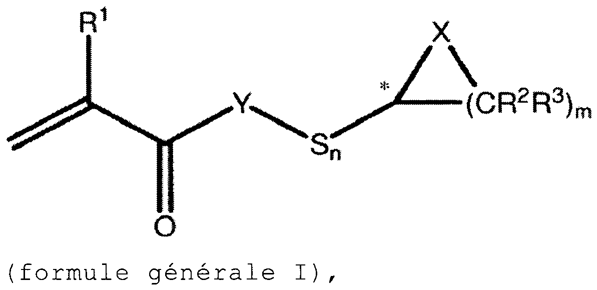 moyennant quoi le polymère possède une capacité d'absorption de l'eau d'au plus 10 % et
R¹, R² et R³ signifient, respectivement, indépendamment l'un de l'autre, de l'hydrogène ou des restes alkyles ;
Y signifie O ou NR⁴ avec R⁴, sélectionné parmi l'hydrogène ou le reste alkyle ;
X signifie O, S, SO ou SO₂ ;
S signifie une unité structurelle, sélectionnée parmi CR⁵₂ et / ou (CR⁵₂CR⁵₂O) ₒCR⁵₂, moyennant quoi tous les R⁵ signifient, respectivement indépendamment l'un de l'autre, de l'hydrogène et / ou des restes alkyles ;
n et o signifient, indépendamment l'un de l'autre, un nombre entier entre 1 et 10 et
m un nombre entier entre 2 et 6.

2. Composition ophtalmologique selon la revendication 1, **caractérisée en ce que** le polymère possède une capacité d'absorption de l'eau d'au maximum 5 %, en particulier d'au maximum 2 % et, de préférence, d'au maximum 1 %.

3. Composition ophtalmologique selon la revendication 1 ou 2, **caractérisée en ce qu'**une proportion en poids du au moins un monomère hydrophobe de la formule générale I est d'entre 2 et 100 pour cent en poids, par rapport au poids total du polymère.

4. Composition ophtalmologique selon l'une des revendications 1 à 3, **caractérisée en ce que** R¹ est de l'hydrogène ou du CH₃-, lorsqu'Y est O ou que R¹ est de l'hydrogène, lorsqu'Y est du NH.

5. Composition ophtalmologique selon l'une des revendications 1 à 4, **caractérisée en ce que** le monomère de la formule générale I est du méthacrylate de tétrahydrofufuryle (THFFMA) et / ou de l'acrylate de tétrahydrofufuryle (THFFA).

6. Composition ophtalmologique selon l'une des revendications 1 à 5, **caractérisée en ce que** le polymère comprend au moins un autre monomère hydrophobe.

7. Composition ophtalmologique selon l'une des revendications 1 à 6, **caractérisée en ce que** le polymère comprend un absorbeur d'UV et / ou au moins un absorbeur de violet.

8. Composition ophtalmologique selon l'une des revendications 1 à 7, **caractérisé en ce que** le polymère comprend au moins un agent de réticulation, moyennant quoi l'agent de réticulation est, en particulier, un absorbeur d'UV et / ou un absorbeur de violet.

9. Composition ophtalmologique selon l'une des revendications 1 à 8, **caractérisée en ce que** le polymère présente un indice de réfraction d'au moins 1,3 et, de préférence, d'au moins 1,5.

10. Composition ophtalmologique selon l'une des revendications 1 à 9, **caractérisée en ce que** le polymère possède une température de transition vitreuse dans la plage entre -10° C et 30° C, en particulier entre 0° C et 30° C et / ou entre 5° C et 20° C.

11. Lentille ophtalmologique, comprenant une composition ophtalmologique selon l'une des revendications 1 à 10.

12. Lentille ophtalmologique selon la revendication 11, **caractérisée en ce que** celle-ci est formée comme une lentille intraoculaire ou comme une lentille de contact.

13. Lentille ophtalmologique selon la revendication 11 ou 12, **caractérisée en ce que** celle-ci présente une teneur en eau d'au maximum 10 %, de préférence d'au maximum 5 %, avantageusement d'au maximum 2 % et de manière particulièrement avantageuse d'au maximum 1 %.

14. Utilisation d'une composition ophtalmologique selon l'une des revendications 1 à 10, destinée à la fabrication d'une lentille ophtalmologique, en particulier d'une lentille de contact ou intraoculaire et / ou d'un implant médical.
